Europäisches Patentamt

European Patent Office

Office européen des brevets

(19)

(11) Veröffentlichungsnummer: **0 370 282**
**A2**

(12) **EUROPÄISCHE PATENTANMELDUNG**

(21) Anmeldenummer: 89120264.0

(51) Int. Cl.⁵: **A61K 7/00**

(22) Anmeldetag: 02.11.89

(30) Priorität: 09.11.88 DE 3837969

(43) Veröffentlichungstag der Anmeldung:
30.05.90 Patentblatt 90/22

(84) Benannte Vertragsstaaten:
ES

(71) Anmelder: **Henkel Kommanditgesellschaft auf Aktien**
**Henkelstrasse 67**
**D-4000 Düsseldorf 13(DE)**

(72) Erfinder: **Möller, Hinrich, Dr.**
**Haydnstrasse 27**
**D-4019 Monheim(DE)**
Erfinder: **Potokar, Matthias, Dr.**
**Spessartstrasse 16**
**D 5628 Heiligenhaus(DE)**

(54) **Entzündungshemmende Wirkstoffe für kosmetische Präparate.**

(57) Derivate der 4-Alkoxybenzoesäure der allgemeinen Formel (I),

4-$R^1$O - $C_6H_4$ - CO - $R^2$ (I)

in der $R^1$ einen Alkylrest mit 1-4 Kohlenstoffatomen und $R^2$ eine Tetrahydrofurfuryloxy-Gruppe, eine Gruppe -O-$(CH_2)_n$-O-$R^3$ oder eine Gruppe -NH-$(CH_2)_n$-$NR^4R^5$ darstellt, wobei n = 2, 3 oder 4 und $R^3$ ein Alkylrest mit 1-4 Kohlenstoffatomen ist sowie $R^4$ und $R^5$ unabhängig voneinander Wasserstoff oder Alkylreste mit 1-4 Kohlenstoffatomen sind oder zusammen mit dem Stickstoffatom ein heterocyclisches Ringsystem darstellen, eignen sich als entzündungshemmende Wirkstoffe zur Herstellung von topischen kosmetischen und pharmazeutischen Mitteln. Die Substanzen weisen keinen oder nur geringen Eigengeruch auf und lassen sich problemlos in übliche pharmazeutische und kosmetische Grundlagen für topische Applikationen einarbeiten.

EP 0 370 282 A2

## Entzündungshemmende Wirkstoffe für kosmetische Präparate

Die Erfindung betrifft die Verwendung von Derivaten der 4-Alkoxy-benzoesäure als entzündungshemmende Wirkstoffe zur Herstellung von topischen pharmazeutischen und kosmetischen Mitteln.

Es ist bekannt, die Wirkung kosmetischer und pharmazeutischer Präparate, insbesondere solcher zur Verwendung auf infolge starker Lichteinstrahlung strapazierter Haut, durch Zugabe entzündungshemmender Substanzen zu erweitern. In der Deutschen Offenlegungsschrift 26 17 817 wurde beispielsweise vorgeschlagen, kosmetischen Präparaten, insbesondere Sonnenschutz- und Sonnenbrandbekämpfungsmitteln, bestimmte Alkoxybenzoesäureester als Entzündungshemmer beizugeben. Wenngleich durch die Zugabe dieser Verbindungen bereits eine deutliche Entzündungshemmung erzielt wird, besteht doch ein Bedürfnis nach Substanzen mit einer höheren Wirksamkeit bei gleichen oder niedrigeren Anwendungskonzentrationen.

Es wurde nun gefunden, daß sich bestimmte Derivate der 4-Alkoxy-benzoesäure der allgemeinen Formel (I),

$$4\text{-}R^1O \text{ - } C_6H_4 \text{ - } CO \text{ - } R^2 \qquad (I)$$

in der $R^1$ einen Alkylrest mit 1-4 Kohlenstoffatomen und $R^2$ eine Tetrahydrofurfuryloxy-Gruppe, eine Gruppe $-O\text{-}(CH_2)_n\text{-}O\text{-}R^3$ oder eine Gruppe $-NH\text{-}(CH_2)_n\text{-}NR^4R^5$ darstellt, wobei $n = 2$, 3 oder 4 und $R^3$ ein Alkylrest mit 1-4 Kohlenstoffatomen ist sowie $R^4$ und $R^5$ unabhängig voneinander Wasserstoff oder Alkylreste mit 1-4 Kohlenstoffatomen sind oder zusammen mit dem Stickstoffatom ein heterocyclisches Ringsystem darstellen, durch eine deutlich größere entzündungshemmende Wirkung auszeichnen. Diese Verbindungen lassen sich problemlos in übliche pharmazeutische und kosmetische Grundlagen für topische Applikationen einarbeiten; sie sind gut kombinierbar mit den anderen üblichen Inhaltsstoffen solcher Zubereitungen und weisen keinen oder nur einen minimalen Eigengeruch auf.

Gegenstand der Erfindung ist somit die Verwendung von Derivaten der 4-Alkoxybenzoesäure der allgemeinen Formel (I) als entzündungshemmende Wirkstoffe zur Herstellung von topischen pharmazeutischen und kosmetischen Mitteln.

Verbindungen der allgemeinen Formel (I) sind bereits als pharmakologische Wirkstoffe bekannt. So beschreibt die niederländische Patentanmeldung 72/4539 u.a. pharmakologische Wirkungen entsprechender Benzoesäureamide. Der japanischen Patentanmeldung 70/32421, zitiert in Chemical Abstracts Bd. 75, Referat 19979w, sind pharmakologische Wirkungen des 4-Methoxy-benzoesäure-2-N,N-diethylaminoethylamid zu entnehmen. In beiden Schriften findet sich jedoch kein Hinweis auf eine entzündungshemmende Wirkung.

Die Verbindungen gemäß Formel (I) sind mit Hilfe bekannter chemischer Umsetzungen aus leicht zugänglichen Ausgangsprodukten herstellbar. Die 4-Alkoxybenzoesäurealkoxyalkylester können beispielsweise aus den 4-Alkoxybenzoesäuren und den Alkoxyalkanolen in Gegenwart eines wasserbindenden Mittels wie Schwefelsäure oder bei azeotroper Entfernung des gebildeten Wassers hergestellt werden. Eines weiteres Herstellungsverfahren ist die Alkoholyse der entsprechenden Benzoesäurechloride. Die 4-Alkoxybenzoesäureaminoamide sind durch Aminolyse der Benzoesäuremethyl- oder -ethylester mit den entsprechenden aminosubstituierten primären Aminen zugänglich. Ein anderes Herstellverfahren ist die Aminolyse der 4-Alkoxybenzoesäurechloride.

In den erfindungsgemäßen Verbindungen steht $R^1$ bevorzugt für Methyl-, Ethyl-, n-Propyl-, iso-Propyl-, n-Butyl-, 2-n-Butyl- und tert.-Butyl-Gruppen. Die Methylgruppe ist dabei besonders bevorzugt. Bevorzugte Gruppen $R^2$ sind die Tetrahydrofurfuryloxy-Gruppe, die Gruppe $-O\text{-}CH_2\text{-}CH_2\text{-}OCH_3$ und die Gruppe $-N\text{-}CH_2\text{-}CH_2\text{-}CH_2\text{-}N(CH_3)_2$. Bevorzugte Gruppen $R^3$ sind Methyl-, Ethyl-, n-Propyl-, iso-Propyl-, n-Butyl-, 2-n-Butyl-Gruppen.

Als Gruppen $NR^4R^5$ werden insbesondere Dimethylamino-, Diethylamino-, Dipropylamino-, Dibutylamino-, n-Butylmethylamino-, Piperidino-, 2-Pipecolino-, 3-Pipecolino-, 4-Pipecolino-, Pyrrolidono-, Pyrrolidino- und Morpholino-Gruppen verwendet.

Erfindungsgemäß ganz besonders bevorzugte Verbindungen der allgemeinen Formel (I) sind Derivate der Anissäure, insbesondere Anissäure-2-methoxyethylester, Anissäure-3-dimethylaminopropylamid und Anissäure-tetrahydrofurfurylester,

Erfindungsgemäß werden die Derivate der 4-Alkoxybenzoesäure der allgemeinen Formel (I) bevorzugt in Mengen von 0,01-10 Gew.-%, insbesondere in Mengen von 0,5-5 Gew.-%, bezogen auf das gesamte Mittel, eingesetzt. Besonders bevorzugt werden die Verbindungen der allgemeinen Formel (I) in Mengen von 2-5 Gew.-%, insbesondere in Mengen von etwa 3 Gew.-%, eingesetzt.

Besonders vorteilhaft ist die Verwendung der Verbindungen der allgemeinen Formel (I) zur Herstellung von Sonnenschutz- und Sonnenbrandbekämpfungsmitteln. Dabei können die 4-Alkoxybenzoesäure-Derivate sowohl in Mitteln, die vor als auch in solchen, die nach einem Sonnenbad auf die Haut aufgebracht werden, Verwendung finden.

Die erfindungsgemäßen 4-Alkoxybenzoesäure-Derivate weisen im Erythem-erzeugenden UV-B-Bereich von etwa 300-320 nm selbst keine Absorptionsbande auf. Zur Herstellung von Mitteln, die vor einem Sonnenbad auf die Haut aufgebracht werden, ist es daher bevorzugt, sie in Kombination mit üblichen UV-Filtersubstanzen einzusetzen.

Geeignete UV-Filtersubstanzen sind beispielsweise:

- Salicylsäurederivate, wie die Ester des Menthols, des Homomenthols, des Fenchols, des Borneols, des Glycerins, des Benzylalkohols, des Phenols, des Ethylenglykols, des 2-Ethylhexanols und des tert.-Butanols sowie das Triäthanolammoniumsalz der Salicylsäure,

- Zimtsäureester, wie p-Methoxy-, p-Amino- und p-Dimethylamino-zimtsäureester, beispielsweise p-Methoxyzimtsäure-3-ethoxyethylester, p-Methoxyzimtsäure-2-ethylhexylester sowie p-Acetamidozimtsäure-isopropylester,

- p-Aminobenzoesäure und ihre Derivate, wie p-Aminobenzoesäureethyl-, -propyl-, -butyl-, -isobutyl-, -monoglycerin- und -dipropylenglykolethylester, p-Dimethylaminobenzoesäureethyl-und -amylester sowie p-Diethylaminobenzoesäureethyl- und amylester,

- substituierte Benzophenone, wie 2,2'-Dihydroxy-4,4'-dimethoxybenzophenon, 2-Hydroxy-4-n-octoxy-benzophenon, 4-Phenylbenzophenon, 2-Hydroxy-4-methoxy-benzophenon-5-sulfonsäure und 4-Phenylbenzophenon-2-carbonsäure-isooctylester,

- Cumarin-Derivate, wie 4-Methylcumarin, 6-Methoxycumarin, 7-Ethylamino-4-methyl-cumarin, 7,8-Dihydroxycumarin, 6,7-Dihydroxycumarin, 7-Hydroxycumarin, 4-Methyl-7-hydroxycumarin und 3-Phenylcumarin, des weiteren 2-Phenylbenzimidazol-5-sulfonsäure, Natrium-3,4-dimethoxyphenylglyoxylat, Butylbenzalaceton, Benzalacetophenon, 3-Toluyliden-D,L-campher, 3-Benzyliden-D,L-campher, 3-(p-Methylbenzyliden)-D,L-campher, Urocaninsäure und deren Salze, Benzimidazol-Derivate, Digalloyltrioleat, Sulfonamide, Hydrazone, Kondensationsprodukte des Tannins mit Ethylenoxid sowie als Naturprodukte Extrakte von Zimtrinde, Ratanhia und Süßholz.

Eine Zusammenstellung geeigneter UV-Filtersubstanzen findet sich auch in der Deutschen Kosmetikverordnung (Anlage 7 zu § 3b).

Die genannten UV-Filtersubstanzen werden üblicherweise in Mengen von 1-10 Gew.-%, bezogen auf das gesamte Mittel, eingesetzt. Mengen von 2-6 Gew.-% sind bevorzugt.

Die erfindungsgemäßen Mittel können als flüssige, pastöse oder feste Zubereitungen formuliert werden, beispielsweise als wäßrige oder alkoholische Lösungen, wäßrige Suspensionen, Emulsionen, Salben, Cremes, Öle, Pulver oder Stifte. In Abhängigkeit von der gewünschten Formulierung können die 4-Alkoxybenzoesäure-Derivate in pharmazeutischen und kosmetischen Grundlagen für topische Applikationen eingearbeitet werden, die als weitere Komponente beispielsweise Ölkomponenten, Fette und Wachse, Emulgatoren, anionische, kationische, ampholytische, zwitterionische und/oder nichtionogene Tenside, niedere ein- und mehrwertige Alkohole, Wasser, Konservierungsmittel, Puffersubstanzen, Verdickungsmittel, Duftstoffe, Farbstoffe und Trübungsmittel enthalten.

Beispiele:

Die nachfolgenden Beispiele dienen zur weiteren Erläuterung der Erfindung, ohne diese darauf zu beschränken.

1. Herstellung der 4-Alkoxybenzoesäure-Derivate

a) Anissäure-2-methoxyethylester

Eine Mischung aus 60,8 g (0,4 Mol) Anissäure, 228 g (3 Mol) Ethylenglykolmonomethylether und 12 g Schwefelsäure wurde 4 Stunden unter Rückfluß erhitzt und anschließend in eine Eis/Wasser-Mischung gegossen. Die organische Phase wurde in Methylenchlorid aufgenommen, je 3 mal mit 10%iger Natriumcarbonat-Lösung und Wasser gewaschen und anschließend mit Natriumsulfat getrocknet. Nach Destillation wurden als Produkt 55 g (66 % d.Th.) an Anissäure-2-methoxyethylester erhalten. Das Produkt hatte einen Siedepunkt von 103-105 °C bei 0,05 mbar und einen Brechungsindex $n_D^{20}$ = 1,5225.

b) Anissäure-3-dimethylaminopropylamid

Zu einer gerührten Lösung von 11,3 g (0,11 Mol) 3-Dimethylamino-propylamin in 200 ml Toluol wurde bei Raumtemperatur eine Lösung von 17,1 g (0,1 Mol) Anissäurechlorid in 100 ml Toluol tropfenweise hinzugegeben. Anschließend wurde die Mischung 2,5 Stunden unter Rückfluß erhitzt. Die erkaltete Suspension wurde mit 10%iger Sodalösung gewaschen und dioe organische Phase nach dem Trocknen mit Natriumsulfat eingedampft. Der Rückstand wurde aus einer Toluol/Hexan-Mischung umkristallisiert. Es wurden 18 g (76 % d.Th.) an Anissäure-3-dimethylaminopropylamid erhalten. Das Produkt wies einen Schmelzpunkt von 63-64 °C auf.

c) Anissäure-2-ethoxyethylester

Die Herstellung erfolgte analog Vorschrift a). Das Produkt wies einen Siedepunkt von 133 °C bei 0,07 mbar auf und hatte einen Brechungsindex $n_D^{20}$ = 1,5149.

d) Anissäure-tetrahydrofurfurylester

Die Herstellung erfolgte analog Vorschrift a). Das Produkt wies einen Siedepunkt von 139-141 °C bei 0,07 mbar auf und hatte einen Brechungsindex $n_D^{20}$ = 1,5373.

e) Anissäure-2-diethylaminoethylamid

Die Herstellung erfolgte analog Vorschrift b). Das Produkt wies einen Siedepunkt von 158 °C bei 0,01 mbar auf und hatte einen Brechungsindex $n_D^{20}$ = 1,5380.

f) Anissäure-3-morpholinopropylamid

Die Herstellung erfolgte analog Vorschrift b). Das Produkt hatte einen Schmelzpunkt von 70-72 °C.

g) Anissäure-3-(2-pipecolino)-propylamid

Die Herstellung erfolgte analog Vorschrift b). Das Produkt hatte einen Brechungsindex $n_D^{20}$ = 1,5482.

h) Anissäure-3-(2-oxo-pyrrolidino)-propylamid

Die Herstellung erfolgte analog Vorschrift b). Das Produkt hatte hatte einen Brechungsindex $n_D^{20}$ = 1,5667.

i) Anissäure-3-diethylaminopropylamid

Die Herstellung erfolgte analog Vorschrift b). Das Produkt hatte einen Siedepunkt von 168 °C bei 0,01 mbar und einen Berechnungsindex $n_D^{20}$ = 1,5363.

j) 4-Ethoxybenzoesäure-tetrahydrofurfurylester

Die Herstellung erfolgte analog Vorschrift a). Das Produkt wies einen Siedepunkt von 132 - 133 °C bei 0,07 mbar auf und hatte einen Brechungsindex $n_D^{20}$ = 1,5311.

2. Testverfahren für die entzündungshemmende Wirkung

4

Zur Bestimmung der entzündungshemmenden Wirkung der Substanzen wurde ein Test an haarlosen Mäusen auf Hautschädigung durch UV-Licht durchgeführt.

Die haarlosen Mäuse wurden am Rücken mit einer UV-Lampe (Ultravitalux-Lampe, OSRAM) auf 45 cm Entfernung 3 Minuten lang bestrahlt, wodurch eine meßbare Hautentzündung (Ödem) ausgelöst wurde. Unmittelbar nach Ende der Bestrahlung wurden die Prüfsubstanzen in Form einer 2,5%igen ethanolischen Lösung mit Hilfe eines Glasstabes aufgetragen. Als Vergleichsversuch wurde bei in gleicher Weise bestrahlten Mäusen eine Nachbehandlung mit dem Trägerstoff Ethanol (Kontrolle) durchgeführt. Nach 30 Stunden wurde der Grad der Ödembildung durch Messung der Hautfaltendicke bestimmt. Durch Vergleich der Veränderung der Hautfaltendicke aufgrund der Bestrahlung bei behandelten und nur mit dem Trägerstoff behandelten Tieren wurde der Grad der Hemmung des Ödems bestimmt.

Es wurden folgende Werte gefunden:

| Substanz | Hemmung des UV-Ödems nach 30 Stunden |
|---|---|
| Anissäure-2-methoxyethylester | 40 % |
| Anissäure-3-dimethylaminopropylamid | 43 % |
| Anissäure-tetrahydrofurfurylester | 43 % |
| 4-Ethoxybenzoesäure-tetrahydrofurfurylester | 50 % |
| Anissäure-propylester (Vergleichssubstanz gemäß DE-OS 26 17 817) | 30 % |

3. Zusammensetzung einer Sonnenschutz-Emulsion

| Komponente: | Gewichtsteile |
|---|---|
| Cutina GMS[(R)1] | 10,0 |
| Erdnußöl | 10,0 |
| Eumulgin[(R)] B 3[2] | 4,0 |
| Glycerin | 5,0 |
| Phenonip[(R)] [3] | 0,2 |
| Wirkstoff gemäß Formel (I) | 3,0 |
| 3-Toluyliden-D,L-campher | 2,0 |
| Farbstoffe, Duftstoffe, Wasser | ad 100,0 |

[1] Glycerinmonostearat DAB 8 (HENKEL)
[2] Cetylstearylalkohol mit ca. 30 Mol Ethylenoxid (HENKEL)
[3] Gemisch aus Hydroxybenzoesäuremethylester, Hydroxybenzoesäureethylester, Hydroxybenzoesäurepropylester, Hydroxybenzoesäurebutylester und Phenoxyethanol (NIPA).

## Ansprüche

1. Verwendung von Derivaten der 4-Alkoxybenzoesäure der allgemeinen Formel (I),

4-$R^1$O - $C_6H_4$ - CO - $R^2$     (I)

in der $R^1$ einen Alkylrest mit 1-4 Kohlenstoffatomen und $R^2$ eine Tetrahydrofurfuryloxy-Gruppe, eine Gruppe -O-$(CH_2)_n$-O-$R^3$ oder eine Gruppe -NH-$(CH_2)_n$-$NR^4R^5$ darstellt, wobei n = 2, 3 oder 4 und $R^3$ ein Alkylrest mit 1-4 Kohlenstoffatomen ist sowie $R^4$ und $R^5$ unabhängig voneinander Wasserstoff oder Alkylreste mit 1-4 Kohlenstoffatomen sind oder zusammen mit dem Stickstoffatom ein heterocyclisches Ringsystem darstellen, als entzündungshemmende Wirkstoffe zur Herstellung von topischen pharmazeutischen und kosmetischen Mitteln.

2. Verwendung nach Anspruch 1, dadurch gekennzeichnet, daß in den Derivaten der 4-Alkoxybenzoesäure der allgemeinen Formel (I) R¹ eine Methylgruppe ist.

3. Verwendung nach einem der Ansprüche 1 oder 2, dadurch gekennzeichnet, daß in den Derivaten der 4-Alkoxybenzoesäure der allgemeinen Formel (I) R² eine Tetrahydrofurfuryloxy-Gruppe, eine Gruppe -O-CH₂-CH₂-OCH₃ oder eine eine Gruppe -N-CH₂-CH₂-CH₂-N(CH₃)₂ ist.

4. Verwendung nach einem der Ansprüche 1 bis 3, dadurch gekennzeichnet, daß die Derivate der 4-Alkoxybenzoesäure der allgemeinen Formel (I) in einer Menge von 0,01 bis 10 Gew.-%, insbesondere von 0,5 bis 5 Gew.-%, bezogen auf das gesamte Mittel, eingesetzt werden.

5. Verwendung nach einem der Ansprüche 1 bis 4, dadurch gekennzeichnet, daß die pharmazeutischen und kosmetischen Mittel Sonnenschutz- und Sonnenbrandbekämpfungsmittel sind.

6. Verwendung nach einem der Ansprüche 1 bis 5, dadurch gekennzeichnet, daß die Derivate der 4-Alkoxybenzoesäure in Kombination mit üblichen UV-Filtersubstanzen eingesetzt werden.

7. Verwendung nach einem der Ansprüche 1 bis 6, dadurch gekennzeichnet, daß die UV-Filtersubstanzen in Mengen von 1 bis 10 Gew.-%, insbesondere 2 bis 6 Gew.-%, bezogen auf das gesamte Mittel, eingesetzt werden.